# EUROPEAN PATENT APPLICATION

(11) **EP 0 770 676 A2**
(43) Date of publication of application: **02.05.1997**
(21) Application number: 96307584.1
(22) Date of filing: 18.10.1996
(51) Int. Cl.: C12N 1/02, C12M 3/06

(54) **Method for treating fermentation broth**

(30) Priority: 23.10.1995 JP 273814/95
(71) Applicant: Ajinomoto Co., Ltd., Tokyo (JP)
(72) Inventor: Tanabe, Toshiya c/o Tech. and Eng. Lab., Kawasaki-ku, Kawasaki-shi, Kanagawa-ken (JP); Ohmori, Kazushige c/o Tech. and Eng. Lab., Kawasaki-ku, Kawasaki-shi, Kanagawa-ken (JP)
(74) Representative: Nicholls, Kathryn Margaret

(57) **Abstract**

The present invention relates to a method of improving a membrane permeation rate when permeating a fermentation broth obtained by culturing a microorganism belonging to the genus Escherichia through a membrane to separate cells is developed. More specifically it relates to a method of treating a fermentation broth, characterized in that a fermentation broth obtained by culturing a microorgasm belonging to the genus Escherichia is heat-treated at from 110 to 200°C with a pH of from 1 to 4.5, and is then filtered through the membrane. The membrane permeation rate of the fermentation broth in the removal of cells therefrom can be improved by from approximately 1.5 to 20 times in comparison to the case where the treatment of the present invention is not conducted. Therefore, the membrane permeation rate can easily be improved and the efficiency of the membrane filtration equipment can be increased.

## Description

The present invention relates to a method in which a membrane fouling substances are preliminarily treated to remarkably improve a permeation rate when removing cells through the membrane, thereby shortening the treatment time and minimizing a size of an membrane equipment .

In recent years, a technology of fermentative production using recombinant strains has advanced, and Escherichia coli (hereinafter abbreviated as "E. coli") which can easily be subjected to the gene manipulation has been often used for this purpose. When a desired product has been excreted outside of strains at the time of the completion of the fermentation, cells are usually removed through membrane separation or centrifugation after sterilization, and the resulting cell-free broth is subjected to the subsequent step. When a desired product is present inside of cells, the cells are milled through freezing or using a homogenizer, a mill or the like, and then the cells and the cell debris are removed through membrane separation or centrifugation.

A membrane for removing cells is generally a microfiltration membrane (hereinafter abbreviated as "MF"), an ultrafiltration membrane (hereinafter abbreviated as "UF") or the like. The membrane separation can completely remove cells, and give a clear cell-free broth compared to the centrifugation. Therefore, the membrane is used to separate cells from a fermentation broth obtained by incubating a microorganism belonging to the genus Brevibacterium or Corynebacterium. However, when a fermentation broth obtained by culturing E. coli strain (hereinafter abbreviated as "an E. coli fermentation broth") is filtered through a membrane in a usual manner, the membrane permeation rate is quite decreased regardless of milling of cells. Thus, it has been found that the use of the membrane in the separation of cells is not practical.

Meanwhile, some methods of improving a membrane permeation rate of a fermentation broth or treating a fermentation broth before subjecting it to the membrane filtration have been known. For example, Japanese Laid-Open Patent Application (Kokai) No. 91,196/1982 describes that when cells and high-molecular substances are separated through an ultrafiltration membrane, a membrane permeation rate is improved by heating an inosine or guanosine fermentation broth at from 90 to 110°C with a pH of from 5.5 to 9.0. Further, Japanese Laid-Open Patent Application (Kokai) No. 78,588/1985 describes that an ultrafiltration membrane permeation rate is improved by heating an amino acid fermentation broth at from 50 to 100°C. Still further, Japanese Laid-Open Patent Application (Kokai) No. 14,795/1984 describes that an amino acid fermentation broth obtained by using cane molasses as carbon source is adjusted to a pH of from 2 to 5, making it possible to improve a rejection ratio of impurities in the ultrafiltration treatment.

However, as stated above, when cells are separated from the E. coli fermentation broth using the membrane, the membrane permeation rate is quite decreased, and this is not practical. The methods disclosed in the above-mentioned documents are not effective for improving the permeation rate in the membrane filtration, and the permeation rate is still low. Thus, these methods have problem in that a large-scaled membrane filtration device is required.

Accordingly, the development of a practical method of improving a membrane permeation rate when filtrating a fermentation broth obtained by culturing a microorganism belonging to the genus Escherichia through a membrane has been in demand.

The present inventors have found that when a fermentation broth obtained by culturing a microorganism belonging to the genus Escherichia is heat-treated at a high temperature after adjusting it to a specific pH range, the microorganism cells themselves or impurities derived from the cells are agglomerated or decomposed, whereby a cake resistance is decreased or concentration polarization is prevented while filtrating the fermentation broth through the membrane. Thus, they have achieved the improvement of the permeation rate in the removal of the cells using the membrane.

That is, the present invention relates to a method for treating a fermentation broth, which comprises filtering a fermentation broth obtained by culturing a microorganism belonging to the genus Escherichia through a membrane to separate cells, characterized in that the fermentation broth is heat-treated at from 110 to 200°C with a pH of from 1 to 4.5, and is then filtered through the membrane.

The microorganism belonging to the genus Escherichia which is used in the present invention may be a wild strain or a mutant. A recombinant strain which is derived through cell fusion or genetic manipulation is also available. An example of microorganisms belonging to the genus Escherichia is Escherichia coli. The desired product produced by this microorganism may be a product which is permeated through a UF membrane or an MF membrane which is used in the present invention, and does not decompose at high temperature with low pH. Examples thereof include amino acids such as lysine, glutamic acid, isoleucine, valine, leucine and phenylalanine; nucleic acids such as inosine and guanosine; organic acids such as lactic acid and malic acid.

The fermentation broth which is used in the present invention can be prepared by culturing the above-mentioned microorganism in an appropriate medium. The medium is not particularly limited, and it may be an ordinary medium containing a carbon source, a nitrogen source, an inorganic ion and optionally an organic nutrient. The fermentation may be conducted by the method described in International Publication No. WO95/16042, Published European Patent Application No. 0685,555 or Japanese Laid-Open Patent Application (Kokai) No. 047,397/1996 under appropriate conditions for growth of the above-mentioned microorganism.

Any carbon source can be used so long as it is applicable to the above-mentioned microorganism. Specific examples of the carbon source include saccharide such as glucose, fructose, sucrose and maltose; organic acids such as fumaric acid, citric acid, acetic acid and propionic acid; and salts thereof.

Any nitrogen source can be used so long as it is applicable to the above-mentioned microorganism. Specific examples of the nitrogen source include inorganic acid ammonium salts such as ammonium sulfate and ammonium chloride; organic acid ammonium salts such as ammonium fumarate and ammonium citrate; nitric acid salts such as sodium nitrate and potassium nitrate; organic nitrogen compounds such as peptone, yeast extract, meat extract and corn steep liquor; and a mixture thereof.

A nutrient source which is used in the ordinary fermentation may be used as required. Examples of the nutrient source include inorganic salts, trace metallic salts and vitamins.

The heat-treatment of a fermentation broth obtained by culturing a microorganism belonging to the genus Escherichia is conducted at from 110 to 200°C, preferably from 110 to 150°C with a pH of from 1 to 4.5, preferably from 2 to 4. The heating time is not particularly limited. It is preferable to conduct the heat-treatment for such a period that the desired product is not denatured or does not decompose. The heat-treatment for 1 minute to 10 minutes is usually preferable. The pH of the fermentation broth can be adjusted with a mineral acid such as hydrochloric acid, sulfuric acid or phosphoric acid.

Moreover, the addition of a polymer agglutination agent such as polyethyleneimine and Sodium polyacrylate to the fermentation is more effective in improving the membrane permeation rate before or after the heat-treatment of the fermentation broth. The amount of the polymer agglutination agent added is between 0.0005 and 0.5% by weight, preferably between 0.001 and 0.05% by weight based on the fermentation broth.

Subsequently, the thus-heat-treated fermentation broth is filtered through a membrane. The membrane which is used in the present invention may be either MF or UF. A flat membrane, a hollow fiber membrane, a tubular membrane or a spiral membrane is available. The material of the membrane may be an organic material such as polysulfone, polyolefin, polyvinylidene difluoride or teflon, or an inorganic material such as ceramics. In the case of UF, a membrane having molecular weight cut off of from 1,000 to 500,000 is appropriate. In the case of MF, a membrane having a pore diameter of from 0.05 to 1 µm is appropriate.

There is no need to adjust the pH of the fermentation broth heat-treated when filtrating it through a membrane. However, the fermentation broth may be adjusted to an appropriate pH depending on the material of the membrane to be used in the filtration. It is not necessary to heat the fermentation broth when filtrating it through a membrane. However, the fermentation broth may be used in the membrane filtration by heating it at from approximately 50 to 60°C and maintaining this temperature in order to obtain a higher permeation rate or prevent rotting.

In this manner, the cells are separated from the fermentation broth, and a clear permeation solution containing a desired product can be obtained.

Examples of the present invention will now be described in detail, by way of example only, with reference to the following figures wherein:

Fig. 1 is a graph showing a change in the membrane permeation rate over the course of time in the removal of cells from a lysine fermentation broth obtained by culturing E. coli.

Fig. 2 is a graph showing a change in the membrane permeation rate over the course of time in the removal of cells from a lysine fermentation broth obtained by culturing E. coli.

Fig. 3 is a graph showing a change in the membrane permeation rate over the course of time in the removal of cells from a leucine fermentation broth obtained by culturing E. coli.

Fig. 4 is a graph showing a change in the membrane permeation rate over the course of time in the removal of cells from a phenylalanine fermentation broth obtained by culturing E. coli.

### Example 1

L-lysine was produced by the method described in Examples of International Publication No. WO95/16042 using B-399/RSFD80 strain obtained by introducing plasmid/RSFD80 into E. coli B-399 strain. The fermentation was conducted at a temperature of 37°C for 48 hours using a medium having the following formulation while stirring the mixture at from 114 to 116 rpm.

### Formulation of the medium for production of L-lysine:

A:

| | |
|---|---|
| (NH₄)₂SO₄ | 16 g/liter |
| KH₂PO₄ | 1 g/liter |
| MgSO₄·7H₂O | 1 g/liter |
| FeSO₄·7H₂O | 0.01 g/liter |
| MnSO₄·5H₂0 | 0.01 g/liter |
| yeast extract (Difco) | 2 g/liter |
| L-methionine | 0.5 g/liter |

The pH was adjusted to 7.0 with KOH, and the mixture was autoclaved at 115°C for 10 minutes (16/20 volume).
B: 20% glucose (which was autoclaved at 115°C for 10 minutes) (4/20 volume)
C: CaCO₃ by Japanese pharmacopeia (which was sterilized with dry heat at 180°C for 2 days) (30 g/liter). A and B were mixed at a ratio of 4:1, and C was added thereto in an amount of 30 g/liter. Antibiotics (100 µg/ml of streptomycin and 5 µg/ml of kanamycin) were added to the mixture.

When the fermentation was completed, the amount of L-lysine produced was 9.2 g/liter calculated as L-lysine hydrochloride. To each 300 ml of this lysine fermentation broth (pH 6.5) was added 98% sulfuric acid to be adjusted to a pH of 4.0 and 2.0, and the fermentation broth were then heat-treated at 120°C. An autoclave was used in the heating. After the temperature of the broth reached 120°C, the broth was kept for 10 minutes at this temperature. As a control, 300 ml of the above-mentioned lysine fermentation broth were subjected to the test for membrane permeation without being heat-treated.

A test for membrane permeation was conducted by mounting two PTTK membranes (UF membranes made by Millipore, U. S. A., molecular weight cut off;30,000) on a Minitan module made by Millipore.

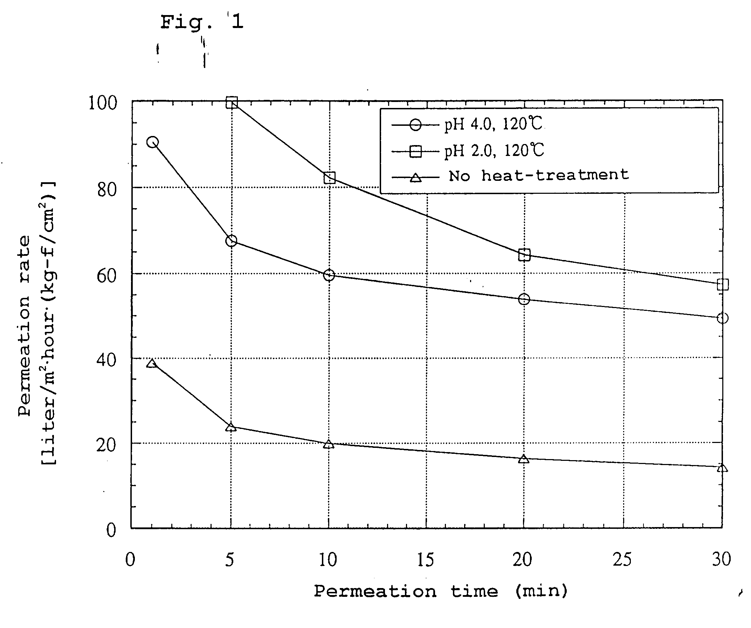

The test for membrane permeation was conducted by feeding the heat-treated fermentation broths to the above-mentioned UF module at a rate of 1,000 ml/min while maintaining the fermentation broth at 50°C and permeating it through the membrane at an average pressure of 0.9 kg-f/cm². The change in the permeation rate was measured over the course of the permeation time of 30 minutes. The results are shown in Fig. 1.

As is clear from Fig. 1, when the fermentation broth was heated at 120°C with a pH of from 2 to 4 in accordance with the method of the present invention, the permeation rate could be improved by approximately three times in comparison to the case in which the heat-treatment was not conducted.

### Comparative Example 1

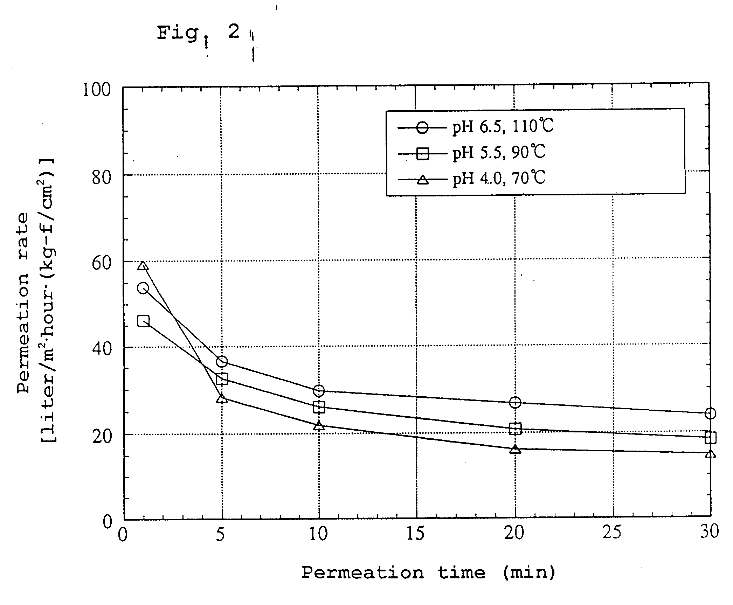

The same E. coli fermentation broth as that used in Example 1 was heat-treated for 10 minutes at 70°C with a pH of 4.0, at 90°C with a pH of 5.5, and at 110°C with a pH of 6.5. The thus-treated fermentation broth was filtered through a membrane in the same manner as in Example 1. At this time, the change in the permeation rate was measured over the course of time, and the results are shown in Fig. 2.

As is apparent from Fig. 2, when the fermentation broth was heat-treated at 70°C with a pH of 4.0, at 90°C with a pH of 5.5 and at 110°C with a pH of 6.5, the permeation rate was, after 30 minutes, approximately 20 liters/m²·hour (kg-f/cm²) which was approximately the same as that in the case where the heat-treatment was not conducted. Thus, the improvement of the permeation rate was not observed.

### Example 2

E. coli FERM -P 5274 strain which belonged to the genus Escherichia, was resistant to β-2-thienyl-alanine and had an ability to produce L-leucine(disclosed in Japanese Laid-Open Patent Application (kokai) No. 34,397/1987) was subjected to a mutation treatment with N-methyl-N'-nitro-N-nitrosoguanigine. Out of the mutated strains, a strain which was resistant to 4-azaleucine was isolated to obtain a strain having an improved ability to produce L-leucine.

The thus-obtained strain was cultured at 31°C for 72 hours with stirring in a medium (containing 5g/dl glucose, (NH₄)₂SO₄ 2.5g/dl, KH₂ PO₄ 0.2g/dl, MgSO₄ ·7H2O 0.1g/dl, yeast extract 0.05g/dl, thiamine hydrochloride 1mg/L, FeSO₄·7H₂O 1mg/dl, MnSO₄·4H₂O 1mg/dl, CaCO₃ 2.5g/dl, pH 7.0). When the fermentation was completed, the concentration of L-leucine accumulated was 3.lg/dl.

98% sulfuric acid was added to this L-leucine fermentation broth to be adjusted to a pH of 4.0 and the fermentation broth were then heat-treated at 120°C. An autoclave was used in the heating. After the temperature of the broth reached 120°C, the broth was kept for 10 minutes at this temperature. As a comparative example, the above-mentioned leucine fermentation broth (pH 4.0) was heat-treated at 60°C for 30 minutes. Two kinds of the heat-treated broths were subjected to the test for membrane permeation.

A test for membrane permeation was conducted by feeding each of the above-treated broths to a Minitan module ( made by Millipore, U. S. A.) mounted two VVLP membranes (MF membranes made by Millipore, pore diameter; 0.1 µm) at a rate of 700 ml/min at an average pressure of 0.9 kg-f/cm². The change of the permeation rate was measured over the course of the time of 80 minutes.

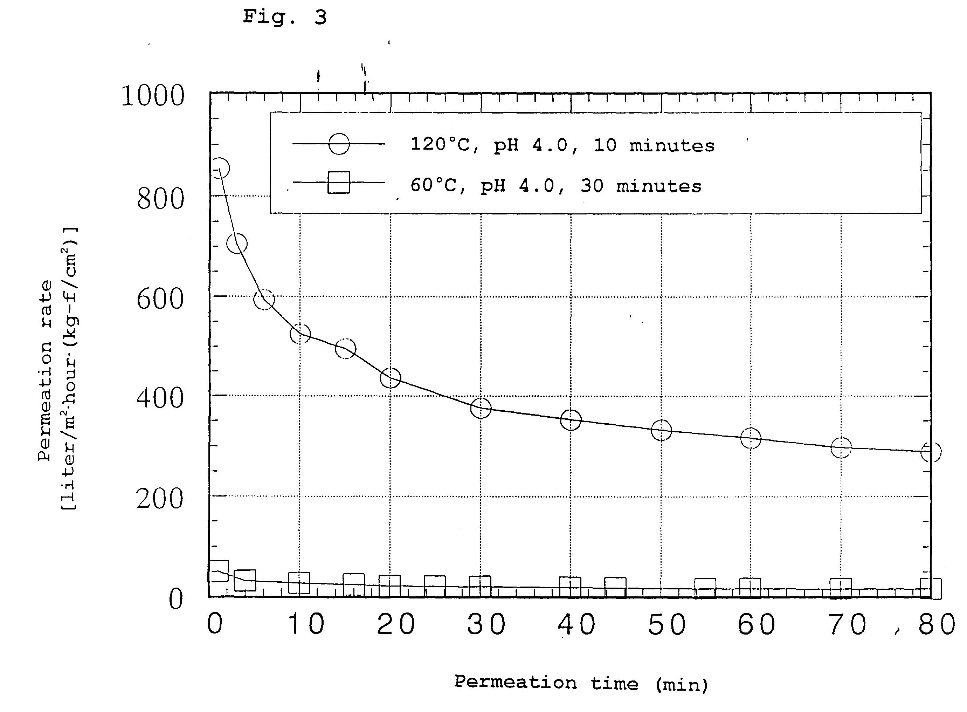

The results are shown in Fig. 3. As is apparent from Fig. 3, it was identified that the membrane permeation rate was increased with the heat-treatment at 120°C in case of L-leucine fermentation broth as well.

### Example 3

L-phenylalanine was produced by culturing E. coli AJ12604 strain described in Published European Patent Application No. 0488,424 at 37°C for 24 hours in a fermentation medium (containing 20 g of glucose, 29.4 g of disodium hydrogen phosphate, 6 g of potassium dihydrogen phosphate, 1 g of sodium chloride, 2 g of ammonium chloride, 10g of sodium citrate, 0.4 g of sodium glutamate, 3 g of magnesium sulfate 7-hydrate, 0.23 g of calsium chloride and 2 mg of thiamine hydrochloride in 1 liter of water). When the fermentation was completed, the concentration of L-phenylalanine accumulated was 3.7 g/liter.

98% sulfuric acid was added to this L-phenylalanine fermentation broth (pH 6.5) to be adjusted to a pH of 4.0 and the fermentation broth were then heat-treated at 120°C for 10 minutes. As a comparative example, the above-mentioned phenylalanine fermentation broth (pH 4.0) was heat-treated at 60°C for 30 minutes. After sodium polyacrylate was added to two kinds of the heat-treated broths (concentration;30 ppm), these broths were subjected to the test for membrane permeation.

A test for membrane permeation was conducted by feeding each of the above-treated broths to UHP-150 module ( made by TOYO ROSHI CO., LTD.) mounted PTFE membranes (MF membranes made by TOYO ROSHI CO., LTD., pore diameter; 1.0 µm) at 60°C at an average pressure of 0.7 kg-f/cm². The change of the permeation rate was measured over the course of the time of 15 minutes.

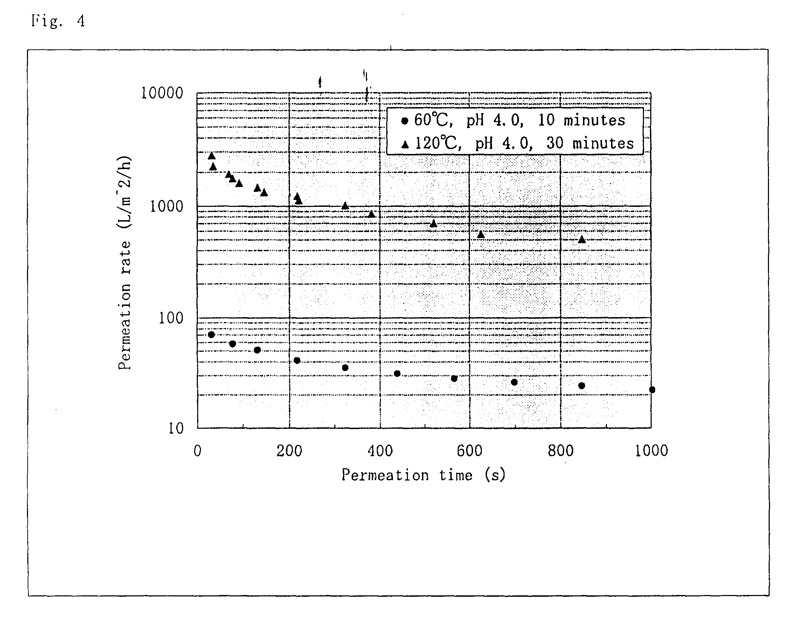

The results are shown in Fig. 4. As is clear from Fig. 4, when the fermentation broth was heated at 120°C with a pH of 4 in accordance with the method of the present invention, the permeation rate could be improved by approximately twenty times in comparison to the case in the heat-treatment at 60°C.

As stated above, in accordance with the method of the present invention, the membrane permeation rate of the fermentation broth can be improved by from approximately 1.5 to 20 times in comparison to the case where the treatment of the present invention is not conducted. Accordingly, a practical membrane permeation rate can be obtained, and the membrane permeation rate of the fermentation broth can easily be improved. Further, it is also possible to increase the efficiency of the membrane filtration equipment.

## Claims

1. A method for treating a fermentation broth, which comprises filtering a fermentation broth obtained by culturing a microorganism belonging to the genus Escherichia through a membrane to separate cells, characterized in that the fermentation broth is heat-treated at from 110 to 200°C with a pH of from 1 to 4.5, and is then filtered through the membrane.

2. The method of claim 1, wherein the membrane used in the filtration through the membrane is an ultrafiltration membrane or a microfiltration membrane.
